# EUROPEAN PATENT APPLICATION

(11) **EP 4 531 054 A1**
(43) Date of publication of application: **02.04.2025**
(21) Application number: 23201025.6
(22) Date of filing: 29.09.2023
(51) Int. Cl.: G16H 40/67, G16H 10/65, G16H 80/00, H04R 25/00

(54) **HEALTH DATA SERVER DEVICE, HEARING SYSTEM AND RELATED METHODS**

(71) Applicant: GN Hearing A/S, 2750 Ballerup (DK)
(72) Inventor: COSTA, Alex Ignatius, DK-2750 Ballerup (DK); BRANDT, Sigurd, DK-2750 Ballerup (DK); NIELSEN, Peter Bredekjær, DK-2750 Ballerup (DK)
(74) Representative: Aera A/S

(57) **Abstract**

A health data server device, the health data server device comprising a memory, one or more interfaces, and one or more processors. The server device is configured to obtain, from an accessory device, accessory data based on hearing data of a hearing device, to obtain, from the accessory device, a first consent to share the accessory data with a health care platform, and to share the accessory data with the healthcare platform in accordance with the first consent.

## Description

The present disclosure relates to a health data server device, a hearing system and related methods including a method of operating a health data server device.

### BACKGROUND

Health conditions of individuals can be difficult to monitor, especially outside of a dedicated healthcare facility such as a hospital. For example, this may be especially difficult as these individuals may be opposed to unsolicited sharing of their data, e.g., health data. However, the condition of an individual may progress and/or decline, potentially unbeknownst to healthcare experts, in the time between visits to a healthcare centre.

### SUMMARY

Accordingly, there is a need for a health data server device, a hearing system and related method with improved consent-based data sharing.

A health data server device is disclosed. The health data server device comprises a memory, one or more interfaces, and one or more processors. The server device is configured to obtain, from an accessory device, accessory data based on hearing data of a hearing device. The server device is configured to obtain, from the accessory device, a first consent to share the accessory data with a health care platform. The server device is configured to share the accessory data with the healthcare platform in accordance with the first consent.

Further, a method of operating a health data server device is disclosed. The method comprises obtaining, from an accessory device, accessory data based on hearing data of a hearing device. The method comprises obtaining, from the accessory device, a first consent to share the accessory data with a health care platform. The method comprises sharing the accessory data with the healthcare platform in accordance with the first consent.

Also disclosed is a hearing system. The hearing system comprises a health data server device as disclosed herein, an accessory device as disclosed herein and a hearing device as disclosed herein.

The present disclosure provides an improved data privacy and improved security of data sharing. The present disclosure enables the sharing of hearing data of a hearing device (such as sensor data from the hearing device) to a healthcare platform via a health data server. The present disclosure allows a user of a hearing device to share hearing data with a healthcare professional in a secure way and while following personal data protection regulations, such as GDPR and HIPAA.

Further, the present disclosure allows the user to give consent remotely, and a healthcare professional to request permission for user data remotely in a secure way.

It is an important advantage of the health data server device that the user may provide consent for a healthcare platform (e.g., used by healthcare experts, e.g., doctors) to obtain via the health data server device accessory data of a hearing device. This may enable a healthcare expert to monitor the health of a user thereby enabling the healthcare expert to provide a higher quality of care to the user of the hearing device. For example, a healthcare expert (e.g., a doctor) may be able to monitor the condition of a user with improved accuracy based on the accessory data shared with the healthcare platform. For example, when a new condition is diagnosed, the healthcare expert may wish to enable health monitoring to allow for optimal disease management. It may be appreciated that the healthcare expert may be able to more accurately choose the data which is relevant by subscribing only to the type of data they find relevant. This may for example combat the increased information overload that exists within existing systems which include plentiful notifications and pop-ups which take time away from the healthcare experts and lead to burn out. In other words, it may give the opportunity for the healthcare experts to be more targeted with their time while getting the optimal benefit from exactly the data they wish to see and nothing more.

The present disclosure allows for improved monitoring of accessory data (e.g., health data) of a user, thereby enabling a healthcare expert to diagnose a patient with increased accuracy and robustness.

The present disclosure may allow to integrate or present data, such as accessory data, in/into existing healthcare platforms, e.g., reducing the number of systems that needs to be visited by healthcare experts and the number of systems a healthcare sector/hospital need to maintain.

### BRIEF DESCRIPTION OF THE DRAWINGS

The above and other features and advantages of the present invention will become readily apparent to those skilled in the art by the following detailed description of exemplary embodiments thereof with reference to the attached drawings, in which:
Fig. 1 schematically illustrates an example hearing system according to the present disclosure and an example health data server device according to the present disclosure, and
Figs. 2A-2B show a flow diagram of an example method according to the disclosure.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

The disclosed hearing system for example comprises hearing device. The hearing device may be configured to be worn at an ear of a user and may be a hearable (e.g., earbuds, headphones, etc) or a hearing aid, wherein the processor is configured to compensate for a hearing loss of a user. In one or more examples or embodiments, the hearing device can be seen as a hearing aid.

The hearing device may be of the behind-the-ear (BTE) type, in-the-ear (ITE) type, in-the-canal (ITC) type, receiver-in-canal (RIC) type or receiver-in-the-ear (RITE) type. The hearing aid may be a binaural hearing aid. The hearing device may comprise a first earpiece and a second earpiece, wherein the first earpiece and/or the second earpiece is an earpiece as disclosed herein.

The hearing device may be configured for wireless communication with one or more devices, such as with another hearing device, e.g., as part of a binaural hearing system, and/or with one or more accessory devices, such as a smartphone and/or a smart watch.

The hearing device optionally comprises an antenna for converting one or more wireless input signals, e.g. a first wireless input signal and/or a second wireless input signal, to antenna output signal(s). The wireless input signal(s) may origin from external source(s), such as spouse microphone device(s), wireless TV audio transmitter, and/or a distributed microphone array associated with a wireless transmitter. The wireless input signal(s) may origin from another hearing device, e.g. as part of a binaural hearing system, and/or from one or more accessory devices.

The hearing device optionally comprises a radio transceiver coupled to the antenna for converting the antenna output signal to a transceiver input signal. Wireless signals from different external sources may be multiplexed in the radio transceiver to a transceiver input signal or provided as separate transceiver input signals on separate transceiver output terminals of the radio transceiver. The hearing device may comprise a plurality of antennas and/or an antenna may be configured to be operate in one or a plurality of antenna modes. The transceiver input signal optionally comprises a first transceiver input signal representative of the first wireless signal from a first external source.

In one or more examples or embodiments, the hearing device comprises a set of microphones. The set of microphones may comprise one or more microphones. The set of microphones comprises a first microphone for provision of a first microphone input signal and/or a second microphone for provision of a second microphone input signal. The set of microphones may comprise N microphones for provision of N microphone signals, wherein N is an integer in the range from 1 to 10. In one or more exemplary hearing devices, the number N of microphones is two, three, four, five or more. The set of microphones may comprise a third microphone for provision of a third microphone input signal.

The hearing device optionally comprises a pre-processing unit. The pre-processing unit may be connected to the radio transceiver for pre-processing the transceiver input signal. The pre-processing unit may be connected the first microphone for pre-processing the first microphone input signal. The pre-processing unit may be connected the second microphone if present for pre-processing the second microphone input signal. The pre-processing unit may comprise one or more A/D-converters for converting analog microphone input signal(s) to digital pre-processed microphone input signal(s).

The hearing device comprises a processor for processing input signals, such as pre-processed transceiver input signal and/or pre-processed microphone input signal(s). The processor provides an electrical output signal based on the input signals to the processor. Input terminal(s) of the processor are optionally connected to respective output terminals of the pre-processing unit. For example, a transceiver input terminal of the processor may be connected to a transceiver output terminal of the pre-processing unit. One or more microphone input terminals of the processor may be connected to respective one or more microphone output terminals of the pre-processing unit.

The hearing device comprises a processor for processing input signals, such as pre-processed transceiver input signal(s) and/or pre-processed microphone input signal(s). The processor is optionally configured to compensate for hearing loss of a user of the hearing device. The processor provides an electrical output signal based on the input signals to the processor. Input terminal(s) of the processor are optionally connected to respective output terminals of the pre-processing unit. For example, a transceiver input terminal of the processor may be connected to a transceiver output terminal of the pre-processing unit. One or more microphone input terminals of the processor may be connected to respective one or more microphone output terminals of the pre-processing unit.

The hearing system, the hearing device, the health data server device, the healthcare platform, and/or the accessory device may be configured for wireless communications via a wireless communication system, such as short-range wireless communications systems, such as Wi-Fi, Bluetooth (such as Auracast), Zigbee, IEEE 802.11, IEEE 802.15, infrared and/or the like. The hearing system, the hearing device, the health data server device, the healthcare platform, and/or the accessory device may be configured for communication via a network such as a global network, e.g. the internet, and/or a local network.

The hearing system, the hearing device and/or the electronic device (such as accessory device) may be configured for wireless communications via a wireless communication system, such as a 3GPP system, such as a 3GPP system supporting one or more of: New Radio, NR, Narrow-band IoT, NB-IoT, and Long Term Evolution - enhanced Machine Type Communication, LTE-M, millimeter-wave communications, such as millimeter-wave communications in licensed bands, such as device-to-device millimeter-wave communications in licensed bands.

In one or more examples or embodiments, the interface of the accessory device and/or the interface of the hearing device comprises one or more of: a Bluetooth interface, Bluetooth low energy interface, and a magnetic induction interface. For example, the interface of the accessory device and/or the interface of the hearing device may comprise a Bluetooth antenna and/or a magnetic interference antenna.

In one or more examples or embodiments, the hearing device as disclosed herein comprises one or more sensors. The one or more sensors may be configured to obtain sensor data. The sensor data may be indicative of the health of the user of the hearing device. It may be appreciated that the hearing data may be based on the sensor data. The one or more sensors may comprise one or more of: a microphone, an optical sensor, a magnetic sensor, a galvanic sensor, a motion sensor, and a capacitive sensor. An example of an optical sensor is a photoplethysmogram (PPG) sensor. An example of a galvanic sensor is an electrocardiogram (ECG) sensor and/or an electroencephalogram (EEG) sensor. A motion sensor may be seen as an inertial measurement unit (IMU). An example of a motion sensor may comprise an accelerometer and/or a gyroscope. A motion sensor may for example comprise a magnetic sensor, such as a magnetometer. An example of a capacitive sensor may comprise a capacitive microphone and/or a temperature sensor.

In one or more hearing systems, hearing devices, and/or electronic devices, the one or more sensors comprise one or more of a body sensor, e.g., a heart rate sensor, a blood pressure sensor, a pulse sensor, a photoplethysmogram (PPG) sensor, an electrocardiogram (ECG) sensor, an electroencephalogram (EEG) sensor, a bioimpedance sensor, and/or a temperature sensor. The one or more sensors of the hearing device may comprise a body sensor being a capacitive and/or conductivity sensor, e.g. to measure and/or determine cognitive decline and/or atrial fibrillation.

The sensor data as disclosed herein may be obtained from one or more of the above sensors. In other words, the sensor data may be generated and/or provided by any one or more of the above mentioned sensors.

A health data server device is disclosed. The health data server device comprises a memory, one or more interfaces, and one or more processors. A health data server device as disclosed herein may be seen as an electronic device e.g., configured to provide a health data service. A health data server device may be seen as a server device for handling health data, such as health data of a plurality of users, e.g., users of hearing devices. The health data server device as disclosed herein may be seen as a secure interface for sharing data between an accessory device of a user and a healthcare platform. For example, the health data server device may be seen as a secure interface for sharing data between a hearing device of a user, e.g., via an accessory device of a user, and a healthcare platform. The health data server device may be a dedicated server device for handling health data of hearing devices and to handle the access and sharing of the health data to third parties, such as the healthcare platform as disclosed herein. The health data server device may be configured to store, manage, and/or securely transmit and/or receive health-related information. The health data server device may be configured to handle the sensitive nature of health data and the need to comply with data protection regulations. The health data server device may allow for medically approved algorithms to analyze data. For example, the health data server device may allow for a configuration that allows for external integration through API's while maintaining secure connection.

The memory of the health data server device may for example be configured to store one or more of: accessory data, hearing data, and consents as disclosed herein,
For example, the health data server device can be seen as a health data server. The terms "health data server device" and "server device" can be used interchangeably.

The server device is configured to obtain, from an accessory device, accessory data based on hearing data of a hearing device, e.g., via the interface of the server device and/or the interface of the accessory device. To obtain accessory data from the accessory device may comprise to obtain, to receive, and/or to retrieve accessory data from the accessory device, e.g., via the interface of the accessory device and/or the one or more interfaces of the health data server device.

An accessory device may be seen as a primary accessory device, such as a primary accessory device of a user. An accessory device may for example be seen as an accessory electronic device. In some examples, the accessory device can comprise memory circuitry, processor circuitry and/or an interface (e.g., a touch screen, one or more wireless interfaces). For example, the accessory device may be an accessory device of a user. The accessory device can for example be a mobile phone, such as a smartphone. In some examples, the accessory device may be configured to run one or more applications. The accessory device may be configured to communicate with the hearing device, e.g., via the wireless interface of the accessory device. The accessory device may be configured to collect and/or gather data, such as hearing data, from the hearing device. The accessory device may be configured to obtain hearing data from the hearing device. To obtain hearing data from the hearing device may comprise to obtain, to receive, and/or to retrieve hearing data from the hearing device, e.g., via the interface of the accessory device and/or the interface of the hearing device. To obtain accessory data from the accessory device may comprise to obtain, to receive, and/or to retrieve accessory data from the accessory device.

The hearing data as disclosed herein may be seen as data obtained by the hearing device. For example, the hearing data comprises data associated with the hearing device of a user. The hearing data may be seen as user data, sensor data, health data, and/or usage data of the user of the hearing device. The hearing data may comprise or be based on one or more of: hearing device settings, audio input data to the hearing device, audio output data from the hearing device, and sensor data of the hearing device. The hearing data may be associated with or indicative of a hearing state and/or a health state of the user. Generally, hearing data may be seen as data obtained from, transmitted from, and/or generated at the hearing device.

Accessory data can for example comprise and/or be based on hearing data obtained (such as received and/or retrieved) by the accessory device, e.g., from a hearing device. The accessory data may for example comprise processed hearing data, such as processed by the accessory device. The processed hearing data is for example based on hearing data of a hearing device. For example, the accessory data may comprise one or more health indicators indicative of a health state of a user. In some examples, the accessory data comprises sensor data, e.g., obtained via a sensor of a hearing device. The accessory data may be derived from the hearing data. For example, the accessory device may be configured to determine or derive health data based on the hearing data. The accessory data may be stored on the accessory device for a certain period of time before being communicated to the health data server device. In one or more examples or embodiments, the accessory data may be deleted from the accessory device after having been communicated to the health data server device. Generally, accessory data may be seen as data obtained from, transmitted from, and/or generated at the accessory device.

In one or more example server devices, the hearing data comprises health data associated with a user of the hearing device. In one or more example server devices, the accessory data is based on the health data.

The user of the hearing device can for example be seen as a person wearing the hearing device. In other words, the hearing data may be indicative of health data associated with the user of the hearing device. The hearing data may comprise health data comprising information indicative of the health of a user of the hearing device. For example, the hearing device is configured to determine or derive health data based on one or more of: hearing device settings, audio input data to the hearing device, audio output data from the hearing device, and sensor data of the hearing device. The health data may be indicative of the health of a user of the hearing device. For example, the health data may comprise data indicative of: heart rate, temperature, blood oxygen, blood pressure, blood sugar levels, respiratory rate, and brain activity etc. The hearing data may for example be obtained by one or more sensors of the hearing device.

In one or more example server devices, the hearing data comprises usage data of the hearing device. In one or more example server devices, the accessory data is based on the usage data.

The usage data for example comprises information indicative of the usage of the hearing device by the user. For example, the hearing device is configured to obtain (such as receive, retrieve, and/or determine) the usage data indicative of the usage of the hearing device. Usage data may be seen as information collected and/or recorded by the hearing device about how the hearing device is used by the user or wearer of the hearing device. Usage data may provide information on the hearing device's performance and/or the user's hearing experience. For example, the usage data may comprise one or more of: average wear time, duration of use, volume settings and/or adjustments, program usage, battery life, speech data, and usage data associated with sensor data, such as accelerometer data. For example, the hearing device is configured to determine or derive usage data based on one or more of: hearing device settings and/or adjustment in settings, audio input data to the hearing device, audio output data from the hearing device, and sensor data of the hearing device. It may be appreciated that the usage data may provide indications on the health of the user of the hearing device. It may be appreciated that the usage data may be related to the health data. In other words, the accessory data, such as health data, may be determined based on the hearing data comprising usage data and sensor data and/or health data. For example, the sensor data may be correlated to the usage data in the time domain. This may allow to determine how much sensor data can be trusted. The usage data may for example be used to set a threshold to determine for how long a hearing device needs to be used in order for the health data server device to have a representative data sample worth sharing with the healthcare platform and in turn with a healthcare expert.

In one or more examples or embodiments, the accessory device is configured to obtain, e.g., via an app of the accessory device and/or a wireless interface of the accessory device and/or a wireless device of the hearing device, the hearing data. In some examples, a user may need to activate an app of the accessory device for the accessory device to obtain (such as receive and/or retrieve) hearing data. In other words, the hearing device may provide (such as transmit) the hearing data to the accessory device, e.g., via an app of the accessory device.

For example, the accessory data may comprise post processed hearing data. Stated differently, in some examples, obtaining, from an accessory device, accessory data may comprise obtaining processed hearing data, such as post processed hearing data. The hearing device, accessory device and/or the health data server device may for example be configured to process, such as post process the hearing data.

In one or more examples, the accessory device may be configured to obtain (such as receive and/or retrieve), from the hearing device, raw (such as unprocessed) hearing data. In one or more examples, the health data server device may be configured to obtain (such as receive and/or retrieve), from the hearing device, e.g., via the accessory device, raw (such as unprocessed) hearing data.

The accessory device may for example, post process the raw hearing data to obtain processed hearing data, and in turn accessory data. The server device may then obtain the post-processed hearing data from the accessory device.

In one or more examples or embodiments, the health data server device may for example be configured to post process the raw hearing data and/or accessory data to obtain processed hearing data and/or processed accessory data, and in turn health data server device data. The healthcare platform may then obtain the post-processed accessory data from the health data server device.

In one or more examples, the accessory data comprises processed data, such as analysed data. In other words, the accessory data may comprise one or more insights (e.g., conclusions) determined based on the hearing data. For example, the accessory device may be configured to process the hearing data.

In some examples, the accessory device, e.g., via an app and using the processor of the accessory device, may be configured to determine one or more insights based on the hearing data. In some examples, the hearing device may be configured to determine one or more insights based on the hearing data. In some examples, the server device may be configured to determine one or more insights based on the hearing data and/or the accessory data. The one or more insights based on the hearing data may for example be provided, e.g., via the server device, to the healthcare platform. The one or more insights may be seen as insights on the health of the user of the hearing device, such as insights on health data.

In some examples, the accessory device may provide (e.g., to a user) the accessory data, e.g., via an app of the accessory device. For example, a user may request (e.g., subscribe to) obtention (such as reception and/or retrieval) of accessory data via an app of the accessory device. In one or more examples or embodiments, a healthcare expert may request (e.g., subscribe to) obtention (such as reception and/or retrieval) of accessory data via the healthcare platform.

The health data server device is configured to obtain, from the accessory device and e.g., via the interface of the accessory device and/or the one or more interfaces of the health data server device, a first consent to share the accessory data with a healthcare platform.

The first consent may for example be provided by a user of the accessory device and/or the hearing device. The first consent may for example be obtained by the accessory device via an app of the accessory device and forwarded to the health data server device and/or the healthcare platform. In one or more examples or embodiments, obtaining the first consent comprises to obtain an indication of a first consent to share the accessory data with a healthcare platform. In other words, the health data server device may not need to obtain the first consent itself, but may receive a message indicating that the user of the hearing device has given consent via the accessory device to share the accessory data with the healthcare platform. The health data server device may then share the accessory data with the healthcare platform in accordance with the message. The first consent may be provided by the user of the hearing device via the accessory device. To share the accessory data may comprise to share health data server device with the healthcare platform. The health data server device may be seen as data generated and/or conveyed by the health data server device.

In the following, an example of how a user gives consent to sharing the accessory data is described. The user may provide the first consent by giving consent to share a data sharing token in the app of the accessory device. The data sharing token may then be transmitted to the healthcare platform by the accessory device and/or the health data server device. The data sharing token may be in the form of a digit code or the like. A healthcare professional may then input the data sharing token into the healthcare platform. After this, the user may be prompted on the accessory device to accept or decline the sharing of accessory data with the healthcare platform, e.g., the accessory device is configured to output a user interface via the display of the accessory device for prompting the user to accept or decline the sharing of accessory data with the healthcare platform. When the user has accepted on the accessory device to share accessory data with the healthcare platform, the accessory device may provide the first consent or the message indicative of the first consent to the health data server device. The health data server device is then configured to share the accessory data with the healthcare platform in accordance with the first consent. In other words, a data link may be established between the health data server device and the healthcare platform based on the first consent. In one or more examples or embodiments, the sharing of the accessory data, such as via the data link, may be performed until the first consent is revoked and/or the healthcare platform stops data subscription.

A consent as disclosed herein, such as the first consent, the second consent, the third consent, and/or the fourth consent, may comprise different type of consents. A consent may for example be a full consent or a partial consent. A partial consent may for example be seen as a time limited consent.

The accessory device is for example configured to obtain consent from a user by providing a selectable options indicative of consent to a user, e.g., via an app of the accessory device. In some examples, the accessory device may provide a "yes" option and a "no" option, e.g., via selectable checkboxes, selectable options in a drop-down menu, etc. For example, when the "yes" option is selected, consent can be seen as granted. For example, when the "no" option is selected, consent can be seen as not granted. In other words, the health data server device may be configured to obtain the first consent from the user via the accessory device.

The healthcare platform for example comprises one or more electronic devices. For example, the healthcare platform comprises one or more databases storing information associated with one or more users of hearing devices (e.g., patients). The healthcare platform for example comprises one or more electronic devices associated with one or more healthcare experts (e.g., doctors, general practitioners, physicians, etc.). For example, an electronic device of the healthcare platform may be a computer of a doctor, where the electronic device may be configured to obtain a user input of a healthcare expert (such as the doctor). The healthcare platform may be seen as an electronic health record system used for the regular tasks associated with healthcare by hospitals and/or general practitioners.

The health data server device is configured to share, e.g., via the one or more interfaces of the health data server device and/or via the interface of the healthcare platform, the accessory data with the healthcare platform in accordance with the first consent.

In one or more examples, to share the accessory data with the healthcare platform comprises to provide (e.g., to transmit) the accessory data to the healthcare platform. In other words, the health data server device may be configured to share the accessory data in response to obtaining the first consent.

For example, a healthcare expert (e.g., a doctor) may be able to monitor the condition of a user with improved accuracy based on the accessory data shared with the healthcare platform.

The present disclosure may enable a healthcare expert to see treatment responses and/or intervene in a timely manner thanks to the access to the shared accessory data. In some examples, a healthcare expert may be enabled to diagnose (e.g., with a medical condition) with an improved accuracy based on the accessory data shared with the healthcare platform.

In one or more example server devices, the server device is configured to obtain, from the healthcare platform and e.g., via the one or more interfaces of the health data server device and/or via the interface of the healthcare platform, a first request for a first permission to share the accessory data with the health care platform.

The first request can for example be seen as request for a first permission, to which a user can provide (such as give) the first consent. The first request can for example be seen as a message provided or conveyed, e.g., by the accessory device, to a user of the accessory device. For example, the first request comprises a message indicative of a request for a first permission to share the accessory data with the healthcare platform. In some examples, the first request comprises a message indicative of a request for permission for the server device to share accessory data with the healthcare platform. The first request may for example be provided by a healthcare expert, such as a doctor, associated with the healthcare platform.

For example, a healthcare expert (e.g., a doctor) may have diagnosed a user (e.g., a patient) of a hearing device with a medical condition or a user showing signs of a condition. The healthcare platform may obtain, e.g., from the doctor, a first request for accessory data of the hearing device of the user. The accessory data requested in the first request is for example associated with the medical condition. For example, the accessory data requested in the first request may be useful for monitoring the medical condition (e.g., health) of the user. In other words, a healthcare expert (e.g., a doctor) may request (e.g., ask) to receive accessory data (e.g., subscribe to an accessory data stream) of a hearing device.

In one or more example server devices, the server device is configured to transmit the first request to the accessory device.

In some examples, upon the server device obtaining the first request from the healthcare platform, the health data server device may provide the first request to the accessory device, such as forward the first request to the accessory device. In other words, the first request may be transmitted from the healthcare platform to the accessory device via the health data server device.

In some examples, upon the accessory device obtaining the first request from the server device, the accessory device may provide the first request to the user (e.g., a user of the accessory device and/or the hearing device). For example, the accessory device may display the first request as a notification (such as a pop-up notification, push notification, etc.). Upon providing the first request to the user, the accessory device may for example obtain a first consent from the user, e.g., in response to the first request.

For example, when the user provides a response indicative of first consent to the first request, the server device has first permission (such as may be permitted to) share the accessory data with the health care platform. Stated differently, when the server device obtains the first consent in response to the first request, the first permission can be seen as granted, e.g., granted based on the first consent.

The first permission can for example be seen as a permission of the server device to share accessory data. For example, based on the first permission, the server device may share accessory data with the healthcare platform.

In one or more example server devices, to obtain a first consent to share the accessory data with a health care platform comprises, in response to the transmitted first request, to obtain, from the accessory device, the first consent to share the accessory data with the health care platform.

In one or more examples, when, in response to the transmitted first request, the server device does not obtain, from the accessory device, a first consent, the server device may refrain from sharing the accessory data with the healthcare platform. In other words, server device can be seen as not having been granted first permission when the first consent is not obtained or when instead of the first consent, a first non-consent or a first refusal to share the accessory data is obtained.

In some examples, the healthcare platform may obtain hearing data via the accessory device. In some examples, the healthcare platform the hearing data via the server device. In other words, sharing the accessory data may comprise forwarding hearing data from the hearing device via the accessory device and the health data server device to the healthcare platform.

In one or more example server devices, the first request comprises a device identifier.

In other words, to obtain the first request may comprise to obtain a device identifier, such as a device identifier associated with a hearing device, a user of a hearing device, and/or an accessory device of a user of a hearing device. For example, a user of a hearing device may have shared his device identifier with a healthcare practitioner, such as a doctor. The healthcare practitioner may then input the device identifier into the healthcare platform. The healthcare platform may be configured to transmit the device identifier with the first request to the health data server device. For example, a doctor has a tab in the user interface of the healthcare platform for device data and access. The doctor may input the device identifier found in the user's mobile app on the accessory device to identify devices to subscribe to. The doctor may then check which data streams to receive. For example, when the device identifier is input by the doctor, the user may get a pop-up up to confirm data sharing with the doctor, allowing data to stream automatically from the health data server device. In one or more example server devices, the server device is configured to obtain accessory data associated with the device identifier. In one or more example server devices, the server device is configured to share the accessory data with the healthcare platform. In other words, the server device may be configured to share the accessory data associated with the device identifier.

The device identifier can for example be seen as a unique identifier indicative of a device, e.g., a hearing device. The device identifier for example comprises a code, e.g., code unique to a given hearing device. For example, the device identifier may comprise a numeric code, an alphabetic code, and/or an alphanumeric code.

In some examples, the device identifier is associated with a hearing device. In some examples, the device identifier is associated with and/or indicative of a user of the hearing device. In some examples, the device identifier is associated with an accessory device of the user. For example, the device identifier may be indicative of an accessory device.

In one or more example server devices, prior to obtaining accessory data, the server device is configured to obtain, from the accessory device, a second consent to obtain the accessory data. In one or more example server devices, the server device is configured to obtain the accessory data in accordance with the second consent.

The second consent can for example be seen as a general consent for the server device to obtain accessory data. The second consent may for example be provided by a user of the accessory device and/or the hearing device. The second consent may for example be obtained by the accessory device via an app of the accessory device.

In one or more examples, when the server device obtains the second consent, e.g., in response to a second request, a second permission can be seen as granted, e.g., granted based on the second consent.

In some examples, the second consent may be provided by a user, e.g., a user of the accessory device and/or the hearing device. For example, when the user provides a user input to the accessory device indicative of a second consent, the server device can be seen as having second permission (such as may be permitted to) obtain the accessory data (e.g., from the accessory device).

In some examples, the user of the hearing device may provide the second consent upon a first-time use of the hearing device (such as the initial use of the hearing device). In one or more examples, the user of the hearing device may provide the second consent prior to a first-time use of the hearing device, e.g., during and/or after the process of transferal of possession of the hearing device, such as during and/or after purchasing the hearing device. The first consent and the second consent do not have any chronological order associated with them, they are merely labels. Therefore, the second consent may be provided before the first consent and vice-versa.

In one or more example server devices, the server device is configured to obtain, from the healthcare platform, a third consent to obtain healthcare platform data from the healthcare platform. In one or more example server devices, the server device is configured to obtain the healthcare platform data from the healthcare platform in accordance with the third consent.

The third consent may for example be provided to or via the healthcare platform by a healthcare expert or practitioner, e.g., a physician, doctor, etc. For example, the healthcare platform may be configured to, upon obtaining a third consent, provide (such as transmit) the healthcare platform data to the server device. Stated differently, when the server device obtains the third consent, a third permission can be seen as granted, e.g., granted based on the third consent.

The third permission can for example be seen as a permission of the server device to share healthcare platform data. For example, based on the third permission, the server device may share healthcare platform data with the healthcare platform. In one or more examples or embodiments, the third consent may be obtained in response to a third request, such as a third request from the user of the accessory device. The third request may be seen as a request from the user of the accessory device to receive data from the healthcare platform.

The healthcare platform data for example comprises data for activation of one or more sensors of the hearing device. Generally, healthcare platform data may be seen as data obtained from, transmitted from, and/or generated at the healthcare platform. Healthcare platform data may for example comprise analyses of the accessory data by a healthcare professional, such as a report and/or a message to the user of the hearing device and/or the accessory device. In other words, the health data server device may enable secure consent-based two-way communication between the healthcare platform and the accessory device, such as between the user of the accessory device and the healthcare practitioner.

In one or more example server devices, the server device is configured to transmit the healthcare platform data to the accessory device. In other words, the health data server device may be configured to transmit the healthcare platform data to the accessory device in response to obtaining the third consent and/or in accordance with the third consent.

In one or more example server devices, the healthcare platform data comprises a sensor activation request for activating a sensor of the hearing device.

The hearing device for example comprises one or more sensors. The one or more sensors of the hearing device can for example be configured to obtain hearing data, e.g., health data. In some examples, the one or more sensors of the hearing device can be configured to obtain heart rate, temperature, blood oxygen, blood pressure, blood sugar levels, respiratory rate, etc. The sensor of the hearing device is for example an internal to or external to the hearing device. The hearing data (e.g., usage data and/or health data) can for example be seen as generated by one or more sensors of the hearing device. In other words, hearing data may comprise sensor data.

The sensor activation request can for example be seen as a request for activation of a sensor, e.g., a sensor of the hearing device. For example, when an accessory device obtains the sensor activation request, the accessory device may be configured to activate a sensor of the hearing device. In other words, upon obtaining (such as receiving and/or retrieving) the sensor activation request from the health data server device, the accessory device may obtain data from an activated sensor of the one or more sensors of the hearing device. Activation of a sensor of the one or more sensors of the hearing device based on the sensor activation request can for example be seen as initiation of a data stream comprising hearing data e.g., to the accessory device. Activation of a sensor may in this context be seen as activating sensor data sharing from the hearing device to the health data server device, e.g., via the accessory device, and in turn from the health data server device to the healthcare platform. For example, activation of a sensor may be seen as activating the recording of sensor data at the hearing device. When a sensor is activated, the hearing device may be configured to include the sensor data in the hearing data. It may be appreciated that the sensor activation request may enable remote secure activation of a sensor of the hearing device and/or of sharing of sensor data from the hearing device by a healthcare professional using the healthcare platform. It may be appreciated that the user of the hearing device may have consented to enable activation of a sensor with a sensor activation request. For example, the user of the hearing device may provide a consent in response to the sensor activation request, e.g., providing a sensor activation permission to the health data server device.

In one or more example server devices, to transmit the healthcare platform data comprises to transmit the sensor activation request to the accessory device. In other words, the healthcare platform data may comprise or be indicative of the sensor activation request. It may be appreciated that the health data server device may convey the sensor activation request from the healthcare platform to the accessory device.

In some examples, upon the accessory device obtaining/receiving the sensor activation request from the server device, the accessory device may provide the sensor activation request to the user (e.g., a user of the accessory device and/or the hearing device). For example, the accessory device may display the sensor activation request as a notification (such as a pop-up notification, push notification, etc.). Upon providing the sensor activation request to the user, the accessory device may for example obtain a sensor activation consent from the user, e.g., in response to the sensor activation request.

In one or more example server devices, the server device is configured to obtain a fourth consent to share the healthcare platform data with the accessory device. In one or more example server devices, the server device is configured to share the healthcare platform data with the accessory device in accordance with the fourth consent.

The fourth consent can for example be seen as a consent, e.g., provided by a user of the accessory device, for the server device to share the healthcare platform data with the accessory device. For example, when the server device obtains, e.g., from the accessory device, the fourth consent, the server device can be seen as having permission to (is permitted to) share the healthcare platform data with the accessory device. For example, the user may provide a user input to the accessory device indicative of a fourth consent.

In one or more example server devices, the server device is configured to obtain, from a secondary accessory device, secondary accessory data.

In some examples, the server device may be configured to obtain (such as receive and/or retrieve), e.g., via an API, secondary accessory data from the secondary accessory device.

The secondary accessory device can be seen as a secondary accessory electronic device. In some examples, the secondary accessory device can comprise memory circuitry, processor circuitry, an interface (e.g., a touch screen), and/or one or more sensors. For example, the secondary accessory device may be an accessory of a user. The secondary accessory device can for example be a smart device and/or a wearable device, such as a smart watch. In some examples, the secondary accessory device may be configured to run one or more applications.

For example, the one or more sensors of the secondary accessory device may be configured to obtain secondary accessory data. The secondary accessory data for example comprises health data. The secondary accessory device is for example configured to obtain, via one or more sensors, health data of a user, e.g., a user of the secondary accessory device, such as a user wearing a smart watch.

In some examples, the secondary accessory device is configured to provide, e.g., via the server device, secondary accessory data to the healthcare platform.

In one or more examples or embodiments, a secondary accessory device consent may be provided by a user of the accessory device, for the server device to share the secondary accessory device data with the accessory device. For example, when the server device obtains, e.g., from the accessory device, the secondary accessory device consent, the server device can be seen as having permission to (is permitted to) share the secondary accessory device data with the accessory device. For example, the user may provide a user input to the accessory device indicative of a secondary accessory device consent. In one or more examples or embodiments, the user may provide a consent on the secondary accessory device to allow the secondary accessory device to share the secondary accessory device data with the accessory device and/or the health data server device.

A hearing system is disclosed. The hearing system comprises a health data server device, such as a health data server device as disclosed herein. The hearing system comprises an accessory device, such as an accessory device as disclosed herein, and a hearing device, such as a hearing device as disclosed herein.

In one or more example hearing systems, the hearing device comprises one or more sensors for provision of sensor data. In one or more example hearing systems, the hearing device is configured to transmit hearing data comprising the sensor data to the accessory device.

Sensor data can for example be seen as data obtained by one or more sensors of the hearing device. In some examples, the sensor data is associated with a user of the hearing device.

In one or more examples, transmitting hearing data from the hearing device to the accessory device comprises transmitting hearing data from the hearing device to the accessory device via a wirelessly and/or via a wired connection. In some examples, the hearing data may be transmitted wirelessly from the hearing device to the accessory device using Near Field Communication, Bluetooth, etc.

In one or more example hearing systems, the hearing system comprises a hearing device data server configured to store hearing data associated with a user of the hearing device.

In one or more examples, the hearing device data server is configured to obtain a consent (such as the consent of a user) from the accessory device to obtain hearing data, e.g., from the hearing device via the accessory device.

In some examples, the accessory device is configured to (e.g., upon obtaining consent of a user) share the hearing data associated with the user of the hearing device to the hearing device data server. In some examples, the hearing device data server can be seen as a hearing device data storage server. In some examples, the hearing device data server may be a part of a data storage facility for example comprising one or more servers configured for storage of hearing data.

It may be appreciated that any of the definitions and terms used in the description in relation to the health data server device, is also applicable to the corresponding feature in the hearing system(s) and the method of operating a health data server device as disclosed herein.

A method of operating a health data server device is disclosed. The method comprises obtaining, from an accessory device, accessory data based on hearing data of a hearing device. The method comprises obtaining, from the accessory device, a first consent to share the accessory data with a health care platform. The method comprises sharing the accessory data with the healthcare platform in accordance with the first consent.

In one or more example methods, the method comprises obtaining, from the healthcare platform, a first request for a first permission to share the accessory data with the health care platform. In one or more example methods, the method comprises transmitting the first request to the accessory device. In one or more example methods, obtaining a first consent to share the accessory data with a health care platform comprises, in response to the transmitted first request, obtaining, from the accessory device, the first consent to share the accessory data with the health care platform.

In one or more example methods, the first request comprises a device identifier. In one or more example methods, the method comprises obtaining accessory data associated with the device identifier. In one or more example methods, the method comprises sharing the accessory data with the healthcare platform.

In one or more example methods, prior to obtaining accessory data, the method comprises obtaining, from the accessory device, a second consent to obtain the accessory data. In one or more example methods, the method comprises obtaining the accessory data in accordance with the second consent.

In one or more example methods, the method comprises obtaining, from the healthcare platform, a third consent to obtain healthcare platform data from the healthcare platform. In one or more example methods, the method comprises obtaining the healthcare platform data from the healthcare platform in accordance with the third consent.

In one or more example methods, the method comprises transmitting the healthcare platform data to the accessory device.

In one or more example methods, the healthcare platform data comprises a sensor activation request for activating a sensor of the hearing device. In one or more example methods, transmitting the healthcare platform data comprises transmitting the sensor activation request to the accessory device.

In one or more example methods, the method comprises obtaining a fourth consent to share the healthcare platform data with the accessory device. In one or more example methods, the method comprises sharing the healthcare platform data with the accessory device in accordance with the fourth consent.

In one or more example methods, the hearing data comprises health data associated with a user of the hearing device. In one or more example methods, the accessory data is based on the health data.

In one or more example methods, the hearing data comprises usage data of the hearing device. In one or more example methods, the accessory data is based on the usage data.

In one or more example methods, the method comprises obtaining, from a secondary accessory device, secondary accessory data.

It is noted that descriptions and features of health data server device functionality, such as health data server device configured to, also apply to methods and vice versa. For example, a description of a health data server device configured to determine also applies to a method, e.g. of operating a health data server device, wherein the method comprises determining and vice versa.

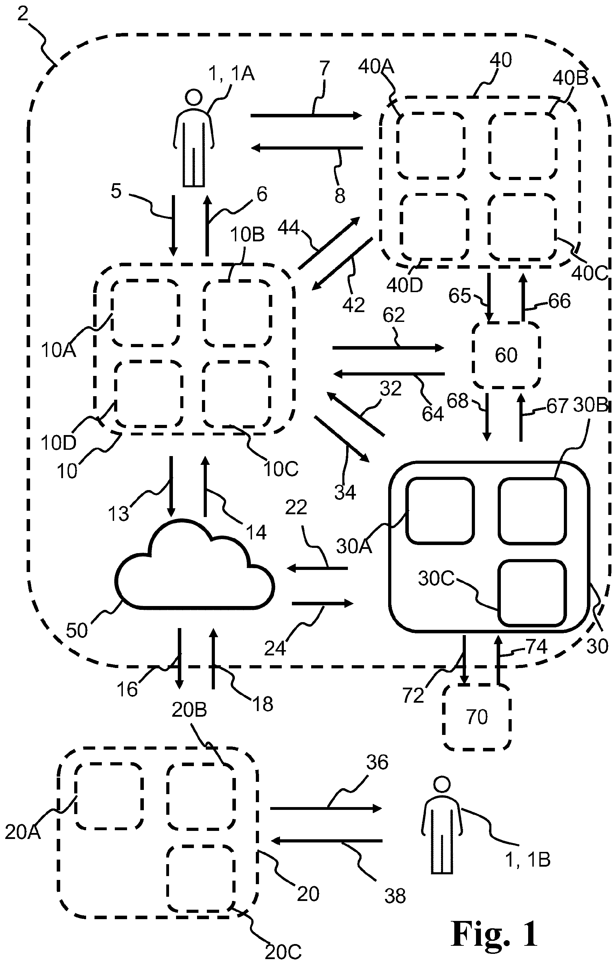

Fig. 1 schematically illustrates an example hearing system, such as a hearing system 2 according to the present disclosure. The hearing system 2 comprises a health data server device 30 according to the present disclosure. The health data server device 30 comprises a memory 30A, one or more interfaces 30B, and one or more processors 30C. The hearing system 2 comprises a hearing device, such as hearing device 40 as disclosed herein. The hearing device 40 may comprise a memory 40A, an interface 40A, a processor 40C, and one or more sensors 40D. The system 2 comprises an accessory device, such as an accessory device 10 as disclosed herein. The accessory device 10 may comprise a memory 10A, an interface 10B, a processor 10C, and one or more sensors 10D. Fig. 1 shows a healthcare platform, such as a healthcare platform 20 as disclosed herein. It may be appreciated that the healthcare platform 20 may not be part of the system 2. The healthcare platform 20 comprises a memory 20A, an interface 20A (such as one or more interfaces), and a processor 20C.

Optionally, the one or more sensors 40D and/or the one or more sensors 10D comprise one or more of: a microphone, an optical sensor, a magnetic sensor, a galvanic sensor, a motion sensor, and a capacitive sensor. In one or more example hearing systems, the hearing device 40 is configured to transmit 42 hearing data comprising the sensor data to the accessory device 10.

Optionally, the interface 10B of the accessory device 10 and/or the interface 40B of the hearing device 40 comprises one or more of: a Bluetooth interface, Bluetooth low energy interface, and a magnetic induction interface. For example, the interface 10B of the accessory device 10 and/or the interface 40B of the hearing device 40 may comprise a Bluetooth antenna and/or a magnetic interference antenna.

The server device 30 is configured to obtain, from the accessory device 10, accessory data based on hearing data of the hearing device 40, e.g., via the interface 30B and/or the interface 10B. To obtain hearing data from the hearing device 40 may comprise to obtain, to receive, and/or to retrieve hearing data from the hearing device 40, e.g., via the interface 40B and/or the interface 10B. To obtain accessory data from the accessory device 10 may comprise to obtain, to receive, and/or to retrieve accessory data from the accessory device 10, e.g., via the interface 30B and/or the interface 10B. In one or more examples or embodiments, to obtain accessory data from the accessory device 10 may comprise to obtain 13, 24 accessory data from the accessory device 10, e.g., via a network 50, such as a global network , e.g. the internet, and/or a local network. In one or more examples or embodiments, to obtain accessory data from the accessory device 10 may comprise to obtain 34 accessory data from the accessory device 10 directly.

In one or more examples or embodiments, the accessory device 10 is configured to obtain 42, e.g., via an app of the accessory device 10 and/or the interface 10B, such as a wireless interface of the accessory device 10, and/or the interface 40B, such as a wireless interface of the hearing device 40, the hearing data. In some examples, a user may need to activate an app of the accessory device 10 for the accessory device 10 to obtain (such as receive and/or retrieve) hearing data. In other words, the hearing device 40 may provide 42 (such as transmit) the hearing data to the accessory device 10, e.g., via an app of the accessory device 10.

In one or more examples, the accessory device 10 may be configured to obtain 42 (such as receive and/or retrieve), from the hearing device 40, raw (such as unprocessed) hearing data. In one or more examples or embodiments, the accessory device 10 is configured to transmit 44 data to the hearing device 40.

Optionally, the user 1, 1A may provide an input 7 (such as user input), such as via the interface 40B, to the hearing device 40. The input 7 may for example be seen as sound input, such as speech input, from the user 1, 1A. The input 7 may comprise measurements, such as measurements on the user 1, 1A, made with the one or more sensors 40D. The hearing data may be based on the input 7 from the user 1, 1A.

Optionally, the hearing device 40 may provide an output 8 to the user 1, 1A (such as a user output), such as via the interface 40B. The output 7 may for example be seen as sound output, such as a processed output of the hearing device 40 in one or more ears of the user 1, 1A.

The hearing data as disclosed herein may be seen as data obtained by the hearing device 40. For example, the hearing data comprises data associated with the hearing device 40 of a user 1, 1A. The hearing data may be seen as user data and/or usage data of the user 1, 1A of the hearing device 40. The hearing data may comprise or be based on one or more of: hearing device settings, audio input data to the hearing device 40, audio output data from the hearing device 40, and sensor data of the hearing device 40, e.g., from the one or more sensors 40D. The hearing data may be associated with or indicative of a hearing state of the user 1, 1A. Generally, hearing data may be seen as data obtained from, transmitted from, and/or generated at the hearing device 40.

Accessory data can for example comprise and/or be based on hearing data obtained (such as received and/or retrieved) by the accessory device 10, e.g., from the hearing device 40. The accessory data may for example comprise processed hearing data, such as processed by the accessory device 10. The processed hearing data is for example based on hearing data of the hearing device 40. For example, the accessory data may comprise one or more health indicators indicative of a health state of the user 1, 1A. In some examples, the accessory data comprises sensor data, e.g., obtained via the one or more sensors 40D of the hearing device 40 and/or via the one or more sensors 10D of the accessory device 10. The accessory data may be derived from the hearing data. The accessory data may be stored on the accessory device 10, such as on the memory 10A, for a certain period of time before being communicated to the health data server device 30. In one or more examples or embodiments, the accessory data may be deleted from the accessory device 10 after having been communicated to the health data server device 30. Generally, accessory data may be seen as data obtained from, transmitted from, and/or generated at the accessory device 10.

In one or more examples or embodiments, the hearing data comprises health data associated with the user 1, 1A of the hearing device 40. In one or more examples, the accessory data is based on the health data.

The user 1, 1A of the hearing device 40 can for example be seen as a person wearing the hearing device 40. In other words, the hearing data is indicative of health data associated with the user 1, 1A of the hearing device 40. The hearing data may comprise health data comprising information indicative of the health of the user 1, 1A of the hearing device 40. For example, the hearing device 40 is configured to determine or derive health data based on one or more of: hearing device settings, audio input data to the hearing device, audio output data from the hearing device 40, and sensor data of the hearing device 40. The health data may be indicative of the health of the user 1, 1A of the hearing device 40. For example, the health data may comprise data indicative of: heart rate, temperature, blood oxygen, blood pressure, blood sugar levels, respiratory rate, and brain activity etc.

In one or more examples or embodiments, the hearing data comprises usage data of the hearing device 40. In one or more examples or embodiments, the accessory data is based on the usage data.

The server device 30 is configured to obtain, from the accessory device 10 (e.g., via the interface 30B and/or the interface 10B), a first consent to share the accessory data with a health care platform, such as healthcare platform 20. The server device 30 is configured to share the accessory data with the healthcare platform 20 in accordance with the first consent. In one or more examples or embodiments, the server device 30 is configured to share the accessory data with the healthcare platform 20 in accordance with the first consent, e.g., via the interface 30B and/or the interface 20B.

The first consent may for example be provided by the user 1, 1A of the accessory device 10 and/or the hearing device 40. The first consent may for example be obtained 5 by the accessory device 10 via an app of the accessory device and forwarded to the health data server device 30 and/or the healthcare platform 20. In one or more examples or embodiments, obtaining the first consent comprises to obtain an indication of a first consent to share the accessory data with the healthcare platform 20. In other words, the health data server device 30 may not need to obtain the first consent itself, but may receive a message indicating that the user 1, 1A of the hearing device 40 has given 5 consent via the accessory device 10 to share the accessory data with the healthcare platform 20. The health data server device 30 may then share the accessory data with the healthcare platform 20 in accordance with the message. The first consent may be provided by the user 1, 1A of the hearing device 40 via the accessory device 10, e.g., with a user input 5. In other words, the health data server device 30 may be configured to obtain the first consent from the user 1, 1A, via the accessory device 10. In one or more examples or embodiments, to obtain the first consent from the accessory device 10 may comprise to obtain 13, 24 the first consent from the accessory device 10, e.g., via a network 50, such as a global network , e.g. the internet, and/or a local network. In one or more examples or embodiments, to obtain the first consent from the accessory device 10 may comprise to obtain 34 the first consent from the accessory device 10 directly.

The healthcare platform 20 for example comprises one or more electronic devices. For example, the healthcare platform 20 comprises one or more databases storing information associated with one or more users of hearing devices (e.g., patients). The healthcare platform for example comprises one or more electronic devices associated with one or more healthcare experts (e.g., doctors, general practitioners, physicians, etc.). For example, an electronic device of the healthcare platform may be a computer of a doctor, where the electronic device may be configured to obtain a user input of a healthcare expert (such as the doctor). In the example of Fig. 1, the healthcare platform 20 is associated with a user 1, 1B, e.g., being a healthcare professional. The healthcare platform 20 may be configured to provide an output 36 to the user 1, 1B of the healthcare platform 20. For example, the healthcare platform 20 may be configured to output 36 the accessory data and/or a representation based on the accessory data to the user 1, 1B.

In one or more examples, to share the accessory data with the healthcare platform 20 comprises to provide 22, 16 (e.g., transmit via a network 50, such as a global network , e.g. the internet, and/or a local network and/or via the interface 30B and/or the interface 20B) the accessory data to the healthcare platform 20. In other words, the health data server device 30 may be configured to share 22, 16 the accessory data in response to obtaining the first consent.

For example, a healthcare expert 1, 1B (e.g., a doctor) may be able to monitor the condition of a user with improved accuracy based on the accessory data shared with the healthcare platform 20.

In some examples, a healthcare expert 1, 1B may be enabled to diagnose (e.g., with a medical condition) with an improved accuracy based on the accessory data shared with the healthcare platform 20.

In one or more example server devices, the server device 30 is configured to obtain 24, from the healthcare platform 20, a first request for a first permission to share the accessory data with the healthcare platform 20. In one or more example server devices, the server device 30 is configured to transmit 22 the first request to the accessory device 10. In one or more example server devices, the server device 30 is configured to, in response to the transmitted first request, to obtain 24, from the accessory device 10, the first consent to share the accessory data with the health care platform 20.

The first request can for example be seen as a message provided or conveyed 6, e.g., by the accessory device 10, to a user 1, 1A of the accessory device 10. For example, the first request comprises a message indicative of a request for a first permission to share the accessory data with the healthcare platform 20. In some examples, the first request comprises a message indicative of a request for permission for the server device 30 to share accessory data with the healthcare platform 20. The first request may for example be provided 38 by the healthcare expert 1, 1B, such as a doctor, associated with the healthcare platform 20.

In one or more example server devices, the first request comprises a device identifier. In one or more example server devices, the server device 30 is configured to obtain 34, 24 accessory data associated with the device identifier. In one or more example server devices, the server device 30 is configured to share the accessory data with the healthcare platform 20.

In other words, to obtain the first request may comprise to obtain a device identifier, such as a device identifier associated with the hearing device 40, the user 1, 1A of the hearing device 40, and/or the accessory device 10 of the user 1, 1A of the hearing device 40. For example, the user 1, 1A of the hearing device 40 may have shared his device identifier with the healthcare practitioner 1, 1B, such as a doctor. The healthcare practitioner 1, 1B may then input 38 the device identifier into the healthcare platform 20. The healthcare platform 20 may be configured to transmit 18 the device identifier with the first request to the health data server device 30.

In one or more example server devices, prior to obtaining accessory data, the server device 30 is configured to obtain 24, 34, from the accessory device 10, a second consent to obtain the accessory data. In one or more example server devices, the server device 30 is configured to obtain 24, 34 the accessory data in accordance with the second consent.

In one or more examples or embodiments, to obtain the second consent from the accessory device 10 may comprise to obtain 13, 24 the second consent from the accessory device 10, e.g., via a network 50, such as a global network , e.g. the internet, and/or a local network. In one or more examples or embodiments, to obtain the second consent from the accessory device 10 may comprise to obtain 34 the second consent from the accessory device 10 directly.

In some examples, the second consent may be provided by the user 1, 1A. For example, when the user provides 5 a user input to the accessory device 10 indicative of a second consent, the server device 30 can be seen as having second permission (such as may be permitted to) obtain the accessory data (e.g., from the accessory device 10).

In one or more example server devices, the server device 30 is configured to obtain 18, 24, from the healthcare platform 20, a third consent to obtain healthcare platform data from the healthcare platform 20. In one or more example server devices, the server device 30 is configured to obtain 18, 24 the healthcare platform data from the healthcare platform 20 in accordance with the third consent.

In one or more examples or embodiments, to obtain the third consent from the healthcare platform 20 may comprise to obtain 18, 24 the third consent from the healthcare platform 20, e.g., via a network 50, such as a global network , e.g. the internet, and/or a local network.

In one or more example server devices, the server device 30 is configured to transmit 22, 32 the healthcare platform data to the accessory device 10. In other words, the health data server device 30 may be configured to transmit 22, 32 the healthcare platform data to the accessory device 10 in response to obtaining the third consent and/or in accordance with the third consent. In one or more examples or embodiments, to transmit the healthcare platform data to the accessory device 10 may comprise to transmit 14, 22, the healthcare platform data to the accessory device 10, e.g., via a network 50, such as a global network , e.g. the internet, and/or a local network. In one or more examples or embodiments, to transmit the healthcare platform data to the accessory device 10 may comprise to transmit 32 the healthcare platform data to the accessory device 10 directly.

In one or more example server devices, the healthcare platform data comprises a sensor activation request for activating a sensor (such as sensor 40D) of the hearing device 40. In one or more example server devices, to transmit 22, 32 the healthcare platform data comprises to transmit 22, 32 the sensor activation request to the accessory device 10. In one or more examples or embodiments, the accessory device 10 is configured to transmit 44 an instruction indicative of the sensor activation to the hearing device 40.

In one or more example server devices, the server device 30 is configured to obtain 24, 34 a fourth consent to share the healthcare platform data with the accessory device 10. In one or more example server devices, the server device 30 is configured to share 22, 32 the healthcare platform data with the accessory device 10 in accordance with the fourth consent. The fourth consent can for example be seen as a consent, e.g., provided by the user 1, 1A of the accessory device 10, for the server device 30 to share the healthcare platform data with the accessory device 10. For example, when the server device 30 obtains 24, 34, e.g., from the accessory device 10, the fourth consent, the server device 30 can be seen as having permission to (is permitted to) share the healthcare platform data with the accessory device 10. For example, the user 1, 1A may provide a user input 5 to the accessory device 10 indicative of a fourth consent.

In one or more example server devices, the hearing data comprises health data associated with the user 1, 1A of the hearing device 40. In one or more example server devices, the accessory data is based on the health data.

In one or more example server devices, the hearing data comprises usage data of the hearing device 40. In one or more example server devices, the accessory data is based on the usage data.

In one or more example server devices, the server device 30 is configured to obtain 74, from a secondary accessory device 70, secondary accessory data. In one or more examples or embodiments, the server device 30 is configured to output or transmit 72 data to the secondary accessory device 70. In one or more examples or embodiments, the server device 30 obtains 24, 34, e.g., from the accessory device 10, a secondary accessory device consent. For example, the server device 30 can be seen as having permission to (is permitted to) share the secondary accessory device data from the secondary accessory device 70 with the accessory device 10. For example, the user 1, 1A may provide a user input to the accessory device 10 and/or the secondary accessory device 70 indicative of a secondary accessory device consent. In one or more examples or embodiments, the user 1, 1A may provide a consent on the secondary accessory device 70 to allow the secondary accessory device 70 to share 74 the secondary accessory device data with the accessory device 10 and/or the health data server device 30. In one or more examples or embodiments, to obtain secondary accessory data from the secondary accessory device 70 may comprise to obtain secondary accessory data from the secondary accessory device 70, e.g., via a network 50, such as a global network , e.g. the internet, and/or a local network. In one or more examples or embodiments, the secondary accessory device 70 may be configured to communicate directly with the electronic device 10, the health data server device 30, and/or the hearing device 40.

In one or more example hearing systems, the hearing system 2 comprises a hearing device data server 60 configured to store hearing data associated with the user 1, 1A of the hearing device 40. In one or more examples, the hearing device data server 60 is configured to obtain 62 a consent (such as the consent of the user 1, 1A) from the accessory device 10 to obtain hearing data, e.g., from the hearing device 40 via the accessory device 10. In one or more examples or embodiments, the hearing device data server 60 is configured to obtain 62 hearing data via the accessory device 10, e.g., via a network 50, such as a global network , e.g. the internet, and/or a local network. In one or more examples or embodiments, the hearing device data server 60 is configured to obtain 65 hearing data directly from the hearing device 40.

In some examples, the accessory device 10 is configured to (e.g., upon obtaining consent of the user 1, 1A) share 64 the hearing data associated with the user 1, 1A of the hearing device 40 to the hearing device data server 60. In some examples, the hearing device data server 60 can be seen as a hearing device data storage server. In some examples, the hearing device data server 60 may be a part of a data storage facility for example comprising one or more servers configured for storage of hearing data.

In one or more examples or embodiments, the hearing device data server 60 is configured to output or transmit 68 data to the health data server device 30. In one or more examples or embodiments, the health data server device 30 is configured to output or transmit 67 data to the the hearing device data server 60. In one or more examples or embodiments, the hearing device data server 60 is configured to output or transmit 66 data to the hearing device 40.

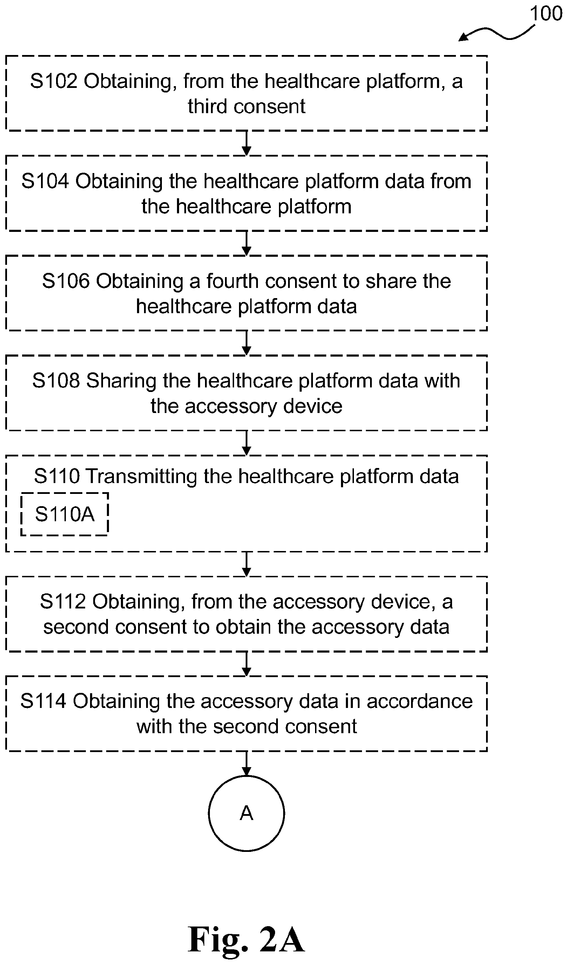

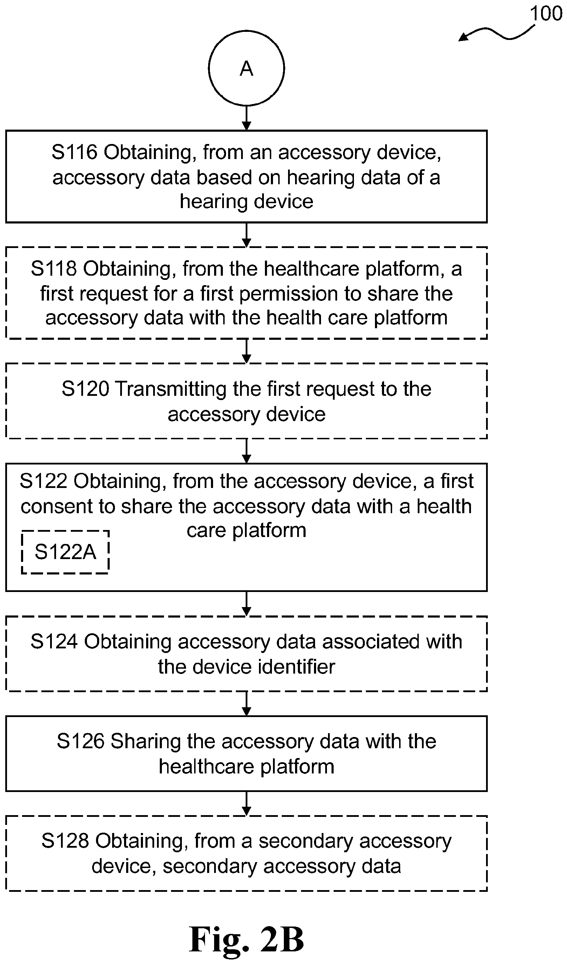

Figs. 2A-2B is a flow diagram of an example method, such as a method 100, according to the present disclosure.

A method of operating a health data server device is disclosed. The method 100, may be performed by a health data server device as disclosed herein, such as health data server device 30 of Fig. 1.

The method 100 comprises obtaining S116, from an accessory device, accessory data based on hearing data of a hearing device.

The method 100 comprises obtaining S 122, from the accessory device, a first consent to share the accessory data with a health care platform.

The method 100 comprises sharing S126 the accessory data with the healthcare platform in accordance with the first consent.

In one or more example methods, the method 100 comprises obtaining S118, from the healthcare platform, a first request for a first permission to share the accessory data with the health care platform. In one or more example methods, the method 100 comprises transmitting S120 the first request to the accessory device. In one or more example methods, obtaining S122 a first consent to share the accessory data with a health care platform comprises, in response to the transmitted first request, obtaining S122A, from the accessory device, the first consent to share the accessory data with the health care platform.

In one or more example methods, the first request comprises a device identifier. In one or more example methods, the method 100 comprises obtaining S124 accessory data associated with the device identifier. In one or more example methods, the method 100 comprises sharing S126 the accessory data with the healthcare platform.

In one or more example methods, prior to obtaining accessory data, the method 100 comprises obtaining S112, from the accessory device, a second consent to obtain the accessory data. In one or more example methods, the method 100 comprises obtaining S114 the accessory data in accordance with the second consent.

In one or more example methods, the method 100 comprises obtaining S102, from the healthcare platform, a third consent to obtain healthcare platform data from the healthcare platform. In one or more example methods, the method 100 comprises obtaining S104 the healthcare platform data from the healthcare platform in accordance with the third consent.

In one or more example methods, the method 100 comprises transmitting S110 the healthcare platform data to the accessory device.

In one or more example methods, the healthcare platform data comprises a sensor activation request for activating a sensor of the hearing device. In one or more example methods, transmitting S110 the healthcare platform data comprises transmitting S110A the sensor activation request to the accessory device.

In one or more example methods, the method 100 comprises obtaining S106 a fourth consent to share the healthcare platform data with the accessory device. In one or more example methods, the method 100 comprises sharing S108 the healthcare platform data with the accessory device in accordance with the fourth consent.

In one or more example methods, the hearing data comprises health data associated with a user of the hearing device. In one or more example methods, the accessory data is based on the health data.

In one or more example methods, the hearing data comprises usage data of the hearing device. In one or more example methods, the accessory data is based on the usage data.

In one or more example methods, the method 100 comprises obtaining S128, from a secondary accessory device, secondary accessory data.

Examples of health data server devices, hearing systems, and methods according to the disclosure are set out in the following items:
Item 1. A health data server device comprising a memory, one or more interfaces, and one or more processors, wherein the server device is configured to:
   obtain, from an accessory device and such as via the one or more interfaces, accessory data based on hearing data of a hearing device;
   obtain, from the accessory device and such as via the one or more interfaces, a first consent to share the accessory data with a health care platform; and
   share, such as via the one or more interfaces, the accessory data with the healthcare platform in accordance with the first consent.
Item 2. The server device according to item 1, wherein the server device is configured to:
   obtain, from the healthcare platform and such as via the one or more interfaces, a first request for a first permission to share the accessory data with the health care platform;
   transmit, such as via the one or more interfaces, the first request to the accessory device; and
   wherein to obtain a first consent to share the accessory data with a health care platform comprises, in response to the transmitted first request, to obtain, from the accessory device, the first consent to share the accessory data with the health care platform.
Item 3. The server device according to item 2, wherein the first request comprises a device identifier, and wherein the server device is configured to obtain accessory data associated with the device identifier, and to share the accessory data associated with the device identifier with the healthcare platform.
Item 4. The server device according to any of previous items, wherein prior to obtaining accessory data, the server device is configured to obtain, from the accessory device, a second consent to obtain the accessory data, and to obtain the accessory data in accordance with the second consent.
Item 5. The server device according to any of the previous items, wherein the server device is configured to obtain, from the healthcare platform, a third consent to obtain healthcare platform data from the healthcare platform, and to obtain the healthcare platform data from the healthcare platform in accordance with the third consent.
Item 6. The server device according to item 5, wherein the server device is configured to transmit the healthcare platform data to the accessory device.
Item 7. The server device according to item 6, wherein the healthcare platform data comprises a sensor activation request for activating a sensor of the hearing device, and wherein to transmit the healthcare platform data comprises to transmit the sensor activation request to the accessory device.
Item 8. The server device according to any of items 6-7, wherein the server device is configured to obtain a fourth consent to share the healthcare platform data with the accessory device, and to share the healthcare platform data with the accessory device in accordance with the fourth consent.
Item 9. The server device according to any of the previous items, wherein the hearing data comprises health data associated with a user of the hearing device, and wherein the accessory data is based on the health data.
Item 10. The server device according to any of the previous items, wherein the hearing data comprises usage data of the hearing device, and wherein the accessory data is based on the usage data.
Item 11. The server device according to any of the previous items, wherein the server device is configured to obtain, from a secondary accessory device, secondary accessory data.
Item 12. A hearing system comprising a health data server device according to any of items 1-11, an accessory device, and a hearing device.
Item 13. The hearing system according to item 12, wherein the hearing device comprises one or more sensors for provision of sensor data, and wherein the hearing device is configured to transmit hearing data comprising the sensor data to the accessory device.
Item 14. The hearing system according to any of items 12-13, wherein the hearing system comprises a hearing device data server configured to store hearing data associated with a user of the hearing device.
Item 15. Method of operating a health data server device, the method comprising:
   obtaining (S116), from an accessory device, accessory data based on hearing data of a hearing device;
   obtaining (S122), from the accessory device, a first consent to share the accessory data with a health care platform; and
   sharing (S126) the accessory data with the healthcare platform in accordance with the first consent.
Item 16. The method of operating a health data server data according to item 15, wherein the method comprises:
   obtaining (S118), from the healthcare platform, a first request for a first permission to share the accessory data with the health care platform;
   transmitting (S120) the first request to the accessory device; and
   wherein obtaining (S122) a first consent to share the accessory data with a health care platform comprises, in response to the transmitted first request, obtaining (S122A), from the accessory device, the first consent to share the accessory data with the health care platform.
Item 17. The method of operating a health data server device according to item 16, wherein the first request comprises a device identifier, and wherein the method comprises obtaining (S124) accessory data associated with the device identifier, and sharing (S126) the accessory data with the healthcare platform.
Item 18. The method of operating a health data server device according to any of items 15-17, wherein prior to obtaining accessory data, method comprises obtaining (S112), from the accessory device, a second consent to obtain the accessory data, and obtaining (S114) the accessory data in accordance with the second consent.
Item 19. The method of operating a health data server device according to any of the items 15-18, wherein the method comprises obtaining (S102), from the healthcare platform, a third consent to obtain healthcare platform data from the healthcare platform, and obtaining (S104) the healthcare platform data from the healthcare platform in accordance with the third consent.
Item 20. The method of operating a health data server device according to item 19, wherein the method comprises transmitting (S110) the healthcare platform data to the accessory device.
Item 21. The method of operating a health data server device according to item 20, wherein the healthcare platform data comprises a sensor activation request for activating a sensor of the hearing device, and wherein transmitting (S110) the healthcare platform data comprises transmitting (S110A) the sensor activation request to the accessory device.
Item 22. The method of operating a health data server device according to any of items 19-20, wherein the method comprises obtaining (S106) a fourth consent to share the healthcare platform data with the accessory device, and sharing (S108) the healthcare platform data with the accessory device in accordance with the fourth consent.
Item 23. The method of operating a health data server device according to any of the previous items 15-22, wherein the hearing data comprises health data associated with a user of the hearing device, and wherein the accessory data is based on the health data.
Item 24. The method of operating a health data server device according to any of the previous items 15-23, wherein the hearing data comprises usage data of the hearing device, and wherein the accessory data is based on the usage data.
Item 25. The method of operating a health data server device according to any of the previous items 15-24, wherein the method comprises obtaining (S128), from a secondary accessory device, secondary accessory data.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering.

Furthermore, the labelling of a first element does not imply the presence of a second element and vice versa.

It may be appreciated that the figures comprise some circuitries or operations which are illustrated with a solid line and some circuitries, components, features, or operations which are illustrated with a dashed line. Circuitries or operations which are comprised in a solid line are circuitries, components, features, or operations which are comprised in the broadest example. Circuitries, components, features, or operations which are comprised in a dashed line are examples which may be comprised in, or a part of, or are further circuitries, components, features, or operations which may be taken in addition to circuitries, components, features, or operations of the solid line examples. It should be appreciated that these operations need not be performed in order presented. Furthermore, it should be appreciated that not all of the operations need to be performed. The example operations may be performed in any order and in any combination. It should be appreciated that these operations need not be performed in order presented. Circuitries, components, features, or operations which are comprised in a dashed line may be considered optional.

Other operations that are not described herein can be incorporated in the example operations. For example, one or more additional operations can be performed before, after, simultaneously, or between any of the described operations.

Certain features discussed above as separate implementations can also be implemented in combination as a single implementation. Conversely, features described as a single implementation can also be implemented in multiple implementations separately or in any suitable sub-combination. Moreover, although features may be described above as acting in certain combinations, one or more features from a claimed combination can, in some cases, be excised from the combination, and the combination may be claimed as any sub-combination or variation of any sub-combination

It is to be noted that the word "comprising" does not necessarily exclude the presence of other elements or steps than those listed. It is to be noted that the words "a" or "an" preceding an element do not exclude the presence of a plurality of such elements. It is to be noted that the term "indicative of' may be seen as "associated with", "related to", "descriptive of", "characterizing", and/or "defining". The terms "indicative of', "associated with" "related to", "descriptive of", "characterizing", and "defining" can be used interchangeably. The term "indicative of' can be seen as indicating a relation. For example, weight data indicative of weight may comprise one or more weight parameters.

It is to be noted that the word "based on" may be seen as "as a function of" and/or "derived from". The terms "based on" and "as a function of" can be used interchangeably. For example, a parameter determined "based on" a data set can be seen as a parameter determined "as a function of" the data set. In other words, the parameter may be an output of one or more functions with the data set as an input.

A function may be characterizing a relation between an input and an output, such as mathematical relation, a database relation, a hardware relation, logical relation, and/or other suitable relations.

It is to be noted that the word "comprising" does not necessarily exclude the presence of other elements or steps than those listed.

It is to be noted that the words "a" or "an" preceding an element do not exclude the presence of a plurality of such elements.

It should further be noted that any reference signs do not limit the scope of the claims, that the exemplary embodiments may be implemented at least in part by means of both hardware and software, and that several "means", "units" or "devices" may be represented by the same item of hardware.

The various exemplary methods, devices, and systems described herein are described in the general context of method steps processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform specified tasks or implement specific abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

Although features have been shown and described, it will be understood that they are not intended to limit the claimed invention, and it will be made obvious to those skilled in the art that various changes and modifications may be made without departing from the spirit and scope of the claimed invention. The specification and drawings are, accordingly to be regarded in an illustrative rather than restrictive sense. The claimed invention is intended to cover all alternatives, modifications, and equivalents.
- 1, 1A, 1B: user
- 2: hearing system
- 5: input
- 6: output
- 7: input
- 8: output
- 10: accessory device
- 10A: memory
- 10B: one or more interfaces
- 10C: processor
- 10D: one or more sensors
- 13: obtain, transmit
- 14: obtain, transmit
- 16: obtain, transmit
- 18: obtain, transmit
- 20: healthcare platform
- 20A: memory
- 20B: one or more interfaces
- 20C: processor
- 22: obtain, transmit
- 24: obtain, transmit
- 30: electronic device, health data server device
- 30A: one or more interfaces
- 30B: memory
- 30C: processor
- 32: obtain, transmit
- 34: obtain, transmit
- 36: obtain, transmit
- 38: obtain, transmit
- 40: hearing device
- 40A: memory
- 40B: one or more interfaces
- 40C: processor
- 40D: one or more sensors
- 42: obtain, transmit
- 44: obtain, transmit
- 50: network
- 60: hearing device data server
- 62: obtain, transmit
- 64: obtain, transmit
- 65: obtain, transmit
- 66: obtain, transmit
- 67: obtain, transmit
- 68: obtain, transmit
- 70: secondary accessory device
- 72: obtain, transmit
- 74: obtain, transmit

## Claims

1. A health data server device comprising a memory, one or more interfaces, and one or more processors, wherein the server device is configured to:
obtain, from an accessory device, accessory data based on hearing data of a hearing device;
obtain, from the accessory device, a first consent to share the accessory data with a health care platform; and
share the accessory data with the healthcare platform in accordance with the first consent.

2. The server device according to claim 1, wherein the server device is configured to:
obtain, from the healthcare platform, a first request for a first permission to share the accessory data with the health care platform;
transmit the first request to the accessory device; and
wherein to obtain a first consent to share the accessory data with a health care platform comprises, in response to the transmitted first request, to obtain, from the accessory device, the first consent to share the accessory data with the health care platform.

3. The health data server device according to claim 2, wherein the first request comprises a device identifier, and wherein the health data server device is configured to obtain accessory data associated with the device identifier, and to share the accessory data associated with the device identifier with the healthcare platform.

4. The health data server device according to any of previous claims, wherein prior to obtaining accessory data, the health data server device is configured to obtain, from the accessory device, a second consent to obtain the accessory data, and to obtain the accessory data in accordance with the second consent.

5. The health data server device according to any of the previous claims, wherein the health data server device is configured to obtain, from the healthcare platform, a third consent to obtain healthcare platform data from the healthcare platform, and to obtain the healthcare platform data from the healthcare platform in accordance with the third consent.

6. The health data server device according to claim 5, wherein the health data server device is configured to transmit the healthcare platform data to the accessory device.

7. The health data server device according to claim 6, wherein the healthcare platform data comprises a sensor activation request for activating a sensor of the hearing device, and wherein to transmit the healthcare platform data comprises to transmit the sensor activation request to the accessory device.

8. The health data server device according to any of claims 6-7, wherein the health data server device is configured to obtain a fourth consent to share the healthcare platform data with the accessory device, and to share the healthcare platform data with the accessory device in accordance with the fourth consent.

9. The health data server device according to any of the previous claims, wherein the hearing data comprises health data associated with a user of the hearing device, and wherein the accessory data is based on the health data.

10. The health data server device according to any of the previous claims, wherein the hearing data comprises usage data of the hearing device, and wherein the accessory data is based on the usage data.

11. The health data server device according to any of the previous claims, wherein the health data server device is configured to obtain, from a secondary accessory device, secondary accessory data.

12. A hearing system comprising a health data server device according to any of claims 1-11, an accessory device, and a hearing device.

13. The hearing system according to claim 12, wherein the hearing device comprises one or more sensors for provision of sensor data, and wherein the hearing device is configured to transmit hearing data comprising the sensor data to the accessory device.

14. The hearing system according to any of claims 12-13, wherein the hearing system comprises a hearing device data server configured to store hearing data associated with a user of the hearing device.

15. Method of operating a health data server device, the method comprising:
obtaining (S116), from an accessory device, accessory data based on hearing data of a hearing device;
obtaining (S122), from the accessory device, a first consent to share the accessory data with a health care platform; and
sharing (S126) the accessory data with the healthcare platform in accordance with the first consent.
